# EUROPEAN PATENT APPLICATION

(11) **EP 4 140 454 A1**
(43) Date of publication of application: **01.03.2023**
(21) Application number: 22192151.3
(22) Date of filing: 25.08.2022
(51) Int. Cl.: A61F 2/64

(54) **ARTIFICIAL KNEE JOINT, PROSTHETIC LIMB, AND JOINT DEVICE**

(30) Priority: 27.08.2021 JP 2021138635
(71) Applicant: Nabtesco Corporation, Tokyo 102-0093 (JP)
(72) Inventor: Okuda, Masahiko, Chiyoda-ku, Tokyo, 102-0093 (JP)
(74) Representative: Grünecker Patent- und Rechtsanwälte PartG mbB

(57) **Abstract**

A multi-articulated link knee joint (100) includes an upper link (16) provided on a thigh part side, a lower link (18) rotatably connected to the upper link (16) via at least one rotation shaft (24, 28), a bearing (207L, 207R) that rotatably supports the rotation shaft (24), an elastic body (208L, 208R) provided between an outer periphery of the rotation shaft (24) and the upper link (16) and elastically deformable in a radial direction of the rotation shaft (24), and a fixing member (202L, 202R) that presses and fixes a pair of left and right front links (20L, 20R) against both end portions of the rotation shaft (24).

## Description

The present invention relates to an artificial knee joint, a prosthetic limb, and a joint device.

### 2. Description of the Related Art

JP H8-229059 A discloses an artificial knee joint or a prosthetic limb worn by a person who has lost a leg due to injury or illness, which includes a cylindrical bearing that rotatably supports a thigh part and a lower leg part around a knee shaft.

In the structure of JP H8-229059 A, when the cylindrical bearing wears along with the rotation of the thigh part and the lower leg part, rattling in the radial direction and/or the axial direction may occur.

The present invention has been made in view of such a situation, and an object of the present invention is to provide an artificial knee joint, a prosthetic limb, and a joint device capable of suppressing occurrence of rattling due to wear along with the rotation around a rotation shaft.

In order to solve the above problems, an artificial knee joint according to an aspect of the present invention includes a thigh joint part provided on a thigh part side, a lower leg part rotatably connected to the thigh joint part via at least one rotation shaft member, a bearing that rotatably supports the rotation shaft member, and an elastic body that is in contact with an outer peripheral surface of the rotation shaft member orthogonal to a rotation shaft, has a longer length from the rotation shaft than the bearing in a direction orthogonal to the rotation shaft, and is elastically deformable in a radial direction of the rotation shaft member.

According to this aspect, even if the contact surface between the rotation shaft member and the bearing wears, the elastic body elastically deformable in the radial direction of the rotation shaft member supports the rotation shaft member, so that the occurrence of rattling in the radial direction can be suppressed.

Another aspect of the present invention is an artificial knee joint. The artificial knee joint includes a thigh joint part provided on a thigh part side, at least one rotation shaft member provided rotatably around a rotation shaft, a lower leg part rotatably connected to the thigh joint part via the rotation shaft member and a pair of links provided at both end portions of the rotation shaft member, and a fixing member that presses and fixes the pair of links against both end portions of the rotation shaft member. At least one of the thigh joint part and the lower leg part is rotatably provided on the outer periphery of the rotation shaft member.

According to this aspect, since the fixing member presses and fixes the pair of links against both end portions of the rotation shaft member, it is possible to suppress occurrence of rattling in the axial direction when at least one of the thigh joint part and the lower leg part rotates on the outer periphery of the rotation shaft member.

Still another aspect of the present invention is a prosthetic limb. The prosthetic limb includes a thigh part, a thigh joint part provided on the thigh part side, a lower leg part rotatably connected to the thigh joint part via at least one rotation shaft member, a bearing that rotatably supports the rotation shaft member, and an elastic body that is in contact with an outer peripheral surface of the rotation shaft member orthogonal to a rotation shaft, has a longer length from the rotation shaft than the bearing in a direction orthogonal to the rotation shaft, and is elastically deformable in a radial direction of the rotation shaft member.

Still another aspect of the present invention is a prosthetic limb. The prosthetic limb includes a thigh part, a thigh joint part provided on the thigh part side, at least one rotation shaft member provided rotatably around a rotation shaft, a lower leg part rotatably connected to the thigh joint part via the rotation shaft member and a pair of links provided at both end portions of the rotation shaft member, and a fixing member that presses and fixes the pair of links against both end portions of the rotation shaft member. At least one of the thigh joint part and the lower leg part is rotatably provided on the outer periphery of the rotation shaft member.

Still another aspect of the present invention is a joint device. The device includes a first member, a second member rotatably connected to the first member via at least one rotation shaft member, a bearing that rotatably supports the rotation shaft member, and an elastic body that is in contact with an outer peripheral surface of the rotation shaft member orthogonal to a rotation shaft, has a longer length from the rotation shaft than the bearing in a direction orthogonal to the rotation shaft, and is elastically deformable in a radial direction of the rotation shaft member.

Still another aspect of the present invention is a joint device. The device includes a first member, at least one rotation shaft member provided rotatably around a rotation shaft, a second member rotatably connected to the first member via the rotation shaft member and a pair of links provided at both end portions of the rotation shaft member, and a fixing member that presses and fixes the pair of links against both end portions of the rotation shaft member. At least one of the first member and the second member is rotatably provided on the outer periphery of the rotation shaft member.

Optional combinations of the aforementioned constituting elements, and implementations of the disclosure in the form of methods, apparatuses, systems, recording mediums, and computer programs may also be practiced as additional modes of the present invention.
Fig. 1 is a side view of a multi-articulated link knee joint;
Fig. 2 is a schematic cross-sectional view of the multi-articulated link knee joint;
Figs. 3A to 3D illustrate a manner in which a knee part is bent in the multi-articulated link knee joint;
Fig. 4 is a functional block diagram of a control device;
Fig. 5 is a schematic cross-sectional view of a multi-articulated link knee joint according to a first configuration example;
Fig. 6 is a schematic cross-sectional view of a multi-articulated link knee joint according to a second configuration example;
Figs. 7A and 7B are schematic cross-sectional views of a multi-articulated link knee joint according to a third configuration example;
Fig. 8 is a schematic cross-sectional view of a multi-articulated link knee joint according to a fourth configuration example;
Figs. 9A to 9D schematically illustrate modification examples of the fourth configuration example;
Figs. 10A and 10B are schematic cross-sectional views of a multi-articulated link knee joint according to a fifth configuration example;
Fig. 11 is a schematic cross-sectional view of a multi-articulated link knee joint according to a sixth configuration example; and
Fig. 12 is a schematic cross-sectional view of a multi-articulated link knee joint according to a seventh configuration example.

The invention will now be described by reference to the preferred embodiments. This does not intend to limit the scope of the present invention, but to exemplify the invention.

There are various types of artificial knee joints, and they are roughly classified into an active type and a passive type. The passive type is further classified into an electronically controlled type and a mechanical type. In the active type, the knee is actively driven to be bent and stretched by an actuator such as a motor. In the electronically controlled passive type, the rotational resistance of the knee is controlled by an electronically controlled hydraulic cylinder or the like according to the bending and stretching motion of the wearer such as walking. In the mechanical passive type, locking of the knee angle or the like is performed by a mechanical system. As will be apparent from the following embodiments, the present invention is applicable to any type of artificial knee joint. The present invention is also applicable to any artificial joint other than the knee.

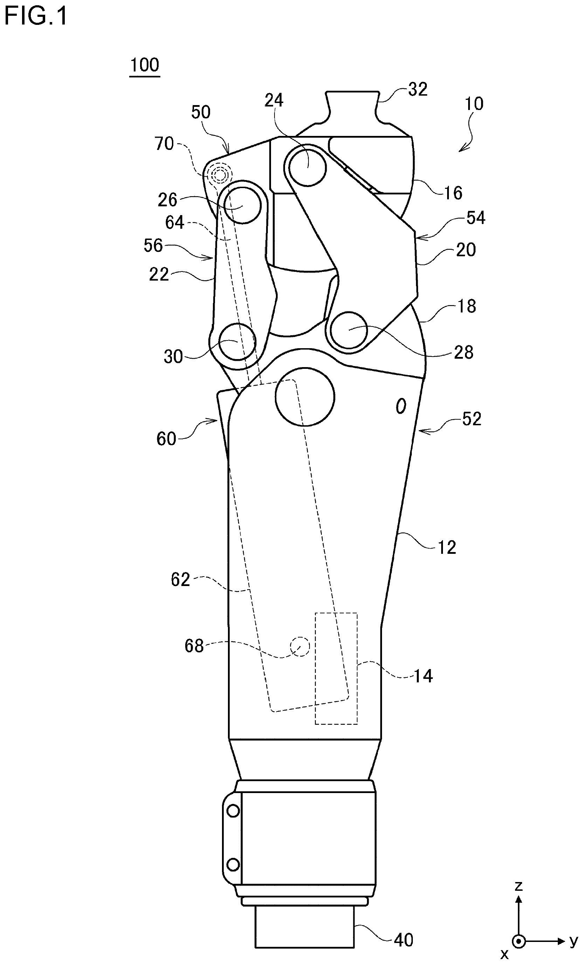

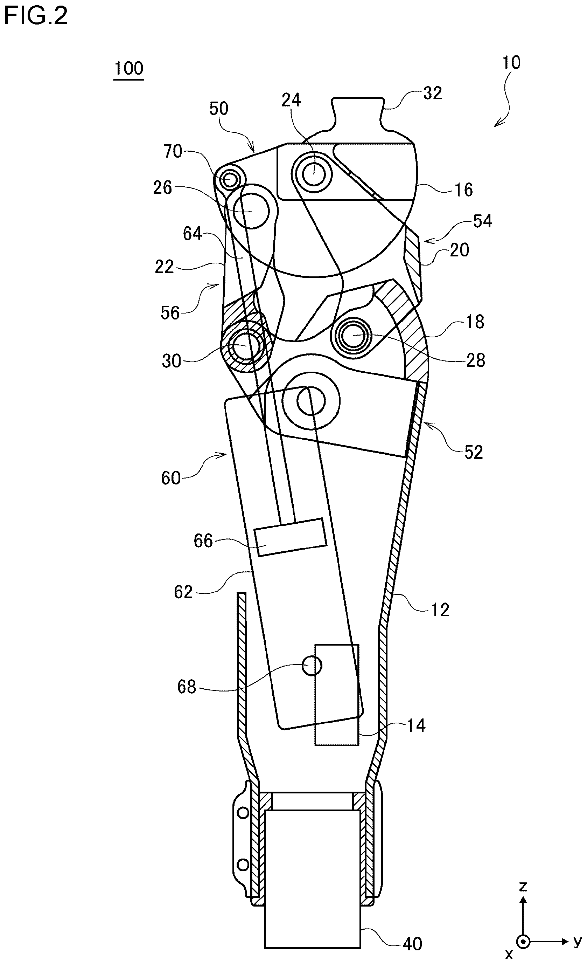

Fig. 1 is a side view of a multi-articulated link knee joint 100 according to an embodiment of the present invention. Fig. 2 is a schematic cross-sectional view of the multi-articulated link knee joint 100 according to the embodiment of the present invention. In the following description, in the xyz orthogonal coordinate system illustrated in each drawing, when provided that the direction parallel to the x-axis is the left-right direction, a positive direction of the x axis is referred to as "left" and a negative direction of the x axis is referred to as "right", when provided that the direction parallel to the y-axis is the front-rear direction, a positive direction of the y axis is referred to as "front" and a negative direction of the y axis is referred to as "rear", and when provided that the direction parallel to the z-axis is the vertical direction, a positive direction of the z axis is referred to as "upper" and a negative direction of the z axis is referred to as "lower".

The multi-articulated link knee joint 100 includes a knee part 10. The knee part 10 is bent by a multi-articulated link mechanism having a plurality of link parts. The multi-articulated link mechanism includes four link parts of an upper link part 50, a lower link part 52, a front link part 54, and a rear link part 56. In the present description, a link and a part that is fixed to the link so as to move together with the link are collectively referred to as a "link part". The upper link part 50 includes an upper link 16 and a thigh joint part 32. The lower link part 52 includes a lower link 18 and a lower leg part 12. The front link part 54 includes a front link 20, and the rear link part 56 includes a rear link 22.

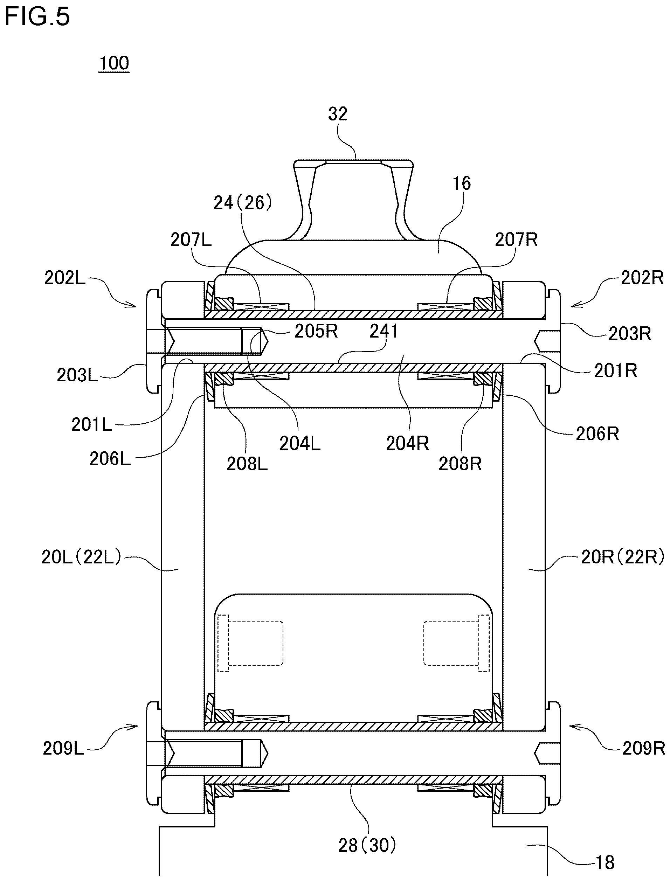

The upper link 16 is provided with a first shaft 24 and a second shaft 26 each as a rotation shaft member or a rotation shaft, and the lower link 18 is provided with a third shaft 28 and a fourth shaft 30 each as a rotation shaft member or a rotation shaft. Each shaft is rotatably provided with the axial direction being parallel to the x axis. The front link 20 is attached to the ends of the first shaft 24 and the third shaft 28. The rear link 22 is attached to the ends of the second shaft 26 and the fourth shaft 30. The upper link 16 is supported by the front link 20 and the rear link 22 and rotates with respect to the lower link 18. In this manner, the upper link 16 to which the thigh joint part 32 is fixed and the lower link 18 to which the lower leg part 12 is fixed are rotatably connected via a plurality of rotation shaft members (that is, a pair of the first shaft 24 and the third shaft 28, or a pair of the second shaft 26 and the fourth shaft 30) and a pair of links (that is, two front links 20 (see Fig. 5) or two rear links 22) connecting both end portions to each other of the plurality of rotation shaft members.

The thigh joint part 32 protruding from the upper link 16 is connected to a socket attached to the thigh part of the user. An angle formed by the direction in which thigh joint part 32 protrudes and the z axis is defined as a bending angle or a knee angle of knee part 10. The bending angle of the knee part 10 can be measured by a knee angle sensor, an inertial sensor, or the like (not illustrated). In Figs. 1 and 2, the bending angle is 0 degrees, and the knee part 10 is fully stretched. The lower leg part 12 is formed in a tubular shape and is fixed below the lower link 18. Furthermore, a foot joint part 40 connected to a foot part constituting the prosthetic limb is provided below the lower leg part 12.

The multi-articulated link knee joint 100 includes a cylinder device 60 as an assisting driver that assists the motion of the knee part 10. The cylinder device 60 includes, for example, an air cylinder or a hydraulic cylinder. The cylinder device 60 includes a cylinder tube 62, a piston rod 64 movable with respect to the cylinder tube 62, and a piston 66 to which the piston rod 64 is fixed and that is movably housed inside the cylinder tube 62. The cylinder device 60 is provided so as to connect the upper link part 50 and the lower link part 52. Specifically, the cylinder tube 62 is rotatably supported by a lower shaft 68 provided in the lower leg part 12 of the lower link part 52, and the piston rod 64 is rotatably supported by an upper shaft 70 provided in the upper link 16 of the upper link part 50. With this configuration, the cylinder device 60 moves or swings in the front-rear direction about the lower shaft 68 according to the rotation of the upper link part 50.

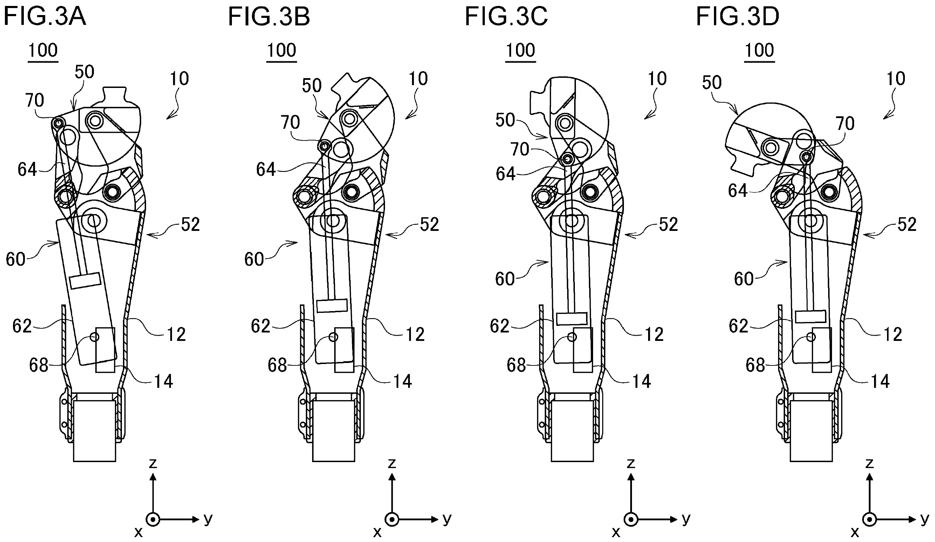

Figs. 3A to 3D illustrate a manner in which a knee part 10 is bent in the multi-articulated link knee joint 100. The bending angles of the knee part 10 illustrated in Figs. 3A to 3D are 0 degrees, 45 degrees, 90 degrees, and 160 degrees, respectively. When the bending angle increases, the front link 20 and the rear link 22 intersect. The upper link 16 rotates while moving backward with respect to the lower link 18. The knee part 10 is bent similarly to the knee joint of the living body by the rotation of the upper link 16.

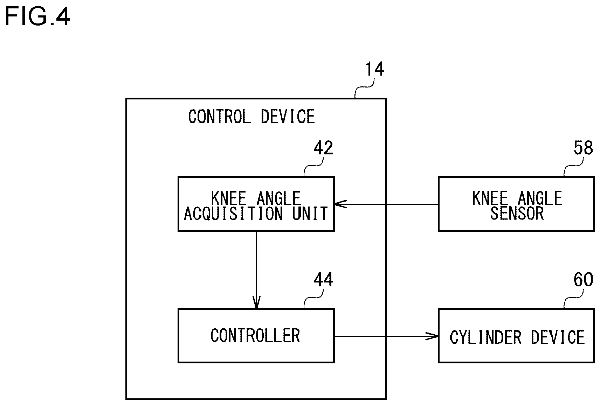

The multi-articulated link knee joint 100 further includes a control device 14. The control device 14 is housed inside the lower leg part 12, and controls the cylinder device 60 according to the bending angle of the knee part 10 measured by a knee angle sensor, an inertial sensor, or the like. Fig. 4 is a functional block diagram of the control device 14. Each functional block illustrated here can be realized by an arithmetic element such as a CPU of a computer in terms of hardware, and is realized by a computer program or the like in terms of software.

The control device 14 includes a knee angle acquisition unit 42 and a controller 44. The knee angle acquisition unit 42 acquires the bending angle of the knee part 10 from a knee angle sensor 58 such as a knee angle sensor or an inertial sensor. The controller 44 controls the cylinder device 60 according to the knee angle acquired by the knee angle acquisition unit 42 to assist the motion of the knee part 10. For example, when the bending angle is close to 0 degrees, the controller 44 controls the cylinder device 60 so as to limit the rotation of the third shaft 28, thereby preventing the knee instability in which the knee part 10 is bent against the intention of the user. In addition, the controller 44 controls the cylinder device 60 to allow the rotation of the third shaft 28 according to the change to be taken in the knee angle during the swing period when the bending angle changes, such as when walking. With this configuration, the lower leg part 12 can swing according to the swinging of each foot at the time of walking, so that the user can walk comfortably.

Fig. 5 is a schematic cross-sectional view of a multi-articulated link knee joint 100 according to a first configuration example taken along a plane including the first shaft 24 and the third shaft 28 or a plane including the second shaft 26 and the fourth shaft 30. In a cross-sectional view taken along a plane including the first shaft 24 and the third shaft 28, a pair of left and right front links 20L and 20R connecting both end portions to each other of both the rotation shaft members 24 and 28 is shown. In a cross-sectional view taken along a plane including the second shaft 26 and the fourth shaft 30, a pair of left and right rear links 22L and 22R connecting both end portions to each other of both the rotation shaft members 26 and 30 is shown. The pair of left and right front links 20L and 20R may be connected in the left-right direction on the front side, and the pair of left and right rear links 22L and 22R may be connected in the left-right direction on the rear side.

In Fig. 5, the lengths in the left-right direction of the upper rotation shaft members 24 and 26 are equal to the lengths in the left-right direction of the lower rotation shaft members 28 and 30, but the lengths of the rotation shaft members 24, 26, 28, and 30 may be different depending on the configuration of the multi-articulated link knee joint 100. Although the pair of left and right front links 20L and 20R and the pair of left and right rear links 22L and 22R sandwich the upper link 16 and the lower link 18 from the outside in Fig. 5, the front links 20L and 20R and/or the rear links 22L and 22R may be sandwiched from the outside by the upper link 16 and/or the lower link 18. Hereinafter, a cross-sectional view taken along a plane including the first shaft 24 and the third shaft 28 will be described.

The first shaft 24, which is an upper rotation shaft member, is formed in a tubular shape (for example, a cylindrical shape) elongated in the left-right direction. The left front link 20L is disposed to face the left end portion of the first shaft 24, and the right front link 20R is disposed to face the right end portion of the first shaft 24. Through holes 201L and 201R communicating with a columnar shaped (for example, cylindrical shaped) internal space 241 of the first shaft 24 are provided in the left front link 20L and the right front link 20R, respectively. A left fixing member 202L serving as a first fixing member is inserted into the left through hole 201L from the left side, and a right fixing member 202R serving as a second fixing member is inserted into the right through hole 201R from the right side.

The left fixing member 202L includes a locking portion 203L provided on the proximal end side (left end side) and having an area larger than the through hole 201L, and a male screw portion 204L extending from the locking portion 203L to the distal end side (right end side). The right fixing member 202R includes a locking portion 203R provided on the proximal end side (right end side) and having an area larger than the through hole 201R, an extending portion 204R extending from the locking portion 203R to the distal end side (left end side), and a female screw portion 205R provided at the distal end portion (left end portion) of the extending portion 204R and engageable with the male screw portion 204L. The left fixing member 202L and the right fixing member 202R are connected to each other inside the first shaft 24 formed in a tubular shape by engagement of the male screw portion 204L and the female screw portion 205R.

Here, when the left fixing member 202L and the right fixing member 202R are firmly fastened, the left front link 20L is strongly pressed and fixed to the left end surface of the first shaft 24 by the locking portion 203L of the left fixing member 202L, and the right front link 20R is strongly pressed and fixed to the right end surface of the first shaft 24 by the locking portion 203R of the right fixing member 202R. In this manner, the left and right front links 20L and 20R and the first shaft 24 are firmly fixed and integrated by the left fixing member 202L and the right fixing member 202R serving as fixing members.

In such a configuration, since a large force is applied to a portion where both end surfaces of the first shaft 24 and the left and right front links 20L and 20R are in contact with each other, each member is formed of a high-strength material. In particular, since the tubular shaped first shaft 24 is easily deformed, it may be formed of a material harder than the left and right front links 20L and 20R. For example, the first shaft 24 is formed of stainless steel, a titanium alloy, or the like, and the left and right front links 20L and 20R are formed of an aluminum alloy or the like. Conversely, in order to prevent the left and right front links 20L and 20R from being depressed at the contact portion with the first shaft 24, the left and right front links 20L and 20R may be formed of a material harder than the first shaft 24. For example, in the left and right front links 20L and 20R mostly made of an aluminum alloy or the like, a high-strength material such as a titanium alloy or hardened steel may be embedded in a washer shape or an annular shape at contact portions with both end surfaces of the first shaft 24 to increase strength. As the hardened steel, martensitic stainless steel such as SUS440C, which hardly generates rust, and stainless steel subjected to an age hardening heat treatment such as SUS630 are suitable. The stainless steel may be subjected to a surface hardening treatment such as a nitriding treatment. At this time, when the contact portions between the first shaft 24 and the left and right front links 20L and 20R are formed of a material having the same composition, there is a possibility that electrical corrosion is promoted, and thus it is preferable to change the composition of one contact portion and the composition of the other contact portion.

The upper link 16 formed integrally with the thigh joint part 32 is rotatably provided on the outer periphery of the first shaft 24. As illustrated in the drawing, the lower portion of the upper link 16 is sandwiched between the pair of left and right front links 20L and 20R, and the length thereof in the left-right direction is shorter than the length of the first shaft 24 in the left-right direction. Therefore, gaps exist between the left end surface of the upper link 16 and the right end surface of the left front link 20L and between the right end surface of the upper link 16 and the left end surface of the right front link 20R. Therefore, the upper link 16 is rotatable around the first shaft 24 in a non-contact manner with the left and right front links 20L and 20R.

A pair of left and right disc springs 206L and 206R each as an end member and an elastic member is provided so as to fill these gaps formed on the outer periphery of both end portions of the first shaft 24. The left disc spring 206L biases the left front link 20L and the upper link 16 in directions away from each other, and the right disc spring 206R biases the right front link 20R and the upper link 16 in directions away from each other. As described above, since the upper link 16 is biased from both sides by the pair of left and right disc springs 206L and 206R, the position in the left-right direction is stabilized regardless of the gap with the left link 20L and the gap with the right front link 20R. Therefore, rattling of the upper link 16 in the left-right direction, that is, the axial direction of the first shaft 24 can be suppressed. The end members that fill the gaps formed on the outer periphery of both end portions of the first shaft 24 are not limited to the disc springs 206L and 206R, and may be plastic or metal washers or coil springs.

A first bearing 207L and a second bearing 207R that rotatably support the first shaft 24 are each provided at a position where the upper link 16 is in contact with the outer periphery of the first shaft 24. The left first bearing 207L is provided on the left end portion side of the first shaft 24, and the right second bearing 207R is provided on the right end portion side of the first shaft 24. Similarly, between the upper link 16 and the outer periphery of the first shaft 24, a first elastic body 208L and a second elastic body 208R that are in contact with the outer periphery of the first shaft 24 and are elastically deformable in the radial direction of the first shaft 24 are provided. In addition, the elastic bodies 208L and 208R are in contact with the outer peripheral surface of the first shaft 24 orthogonal to the rotation shaft, and have a longer length from the rotation shaft than the bearings 207L and 207R in the direction orthogonal to the rotation shaft. Here, while the elastic bodies 208L and 208R are in contact with the outer periphery of the first shaft 24 by elastic deformation, minute gaps of about several tens of microns exist between the bearings 207L and 207R and the outer periphery of the first shaft 24. Therefore, the elastic bodies 208L and 208R protrude from the bearings 207L and 207R toward the center of the first shaft 24.

The left first elastic body 208L is provided in contact with the outer periphery of the left end portion of the first shaft 24, and the right second elastic body 208R is provided in contact with the outer periphery of the right end portion of the first shaft 24. The left first elastic body 208L is provided closer to the left end portion of the first shaft 24 than the left first bearing 207L, and the right second elastic body 208R is provided closer to the right end portion of the first shaft 24 than the right second bearing 207R. The elastic bodies 208L and 208R are formed of, for example, rubber, resin, elastomer, or the like. Instead of the pair of left and right bearings 207L and 207R, one bearing may be provided at the center of the first shaft 24 in the axial direction.

The pair of left and right first bearings 207L and second bearings 207R may be configured as rolling bearings, but are preferably configured as plain bearings made of plastic or copper alloy, which are inexpensive, in order to reduce the cost of the multi-articulated link knee joint 100. In this case, when the bearings 207L and 207R wear due to the rotation of the upper link 16 around the first shaft 24, a gap in the radial direction is generated between the bearing and the outer periphery of the first shaft 24. However, since the elastic bodies 208L and 208R provided adjacent to the bearings 207L and 207R are elastically deformed in the radial direction, the contact state between the upper link 16 and the first shaft 24 is maintained via the elastic bodies 208L and 208R, it is possible to suppress rattling in the radial direction when the upper link 16 rotates around the first shaft 24 regardless of the gap in the radial direction caused by wear of the bearings 207L and 207R.

The pair of left and right elastic bodies 208L and 208R is elastically deformable in the axial direction in contact with the disc springs 206L and 206R as end members provided at both end portions of the first shaft 24. Here, the pair of left and right elastic bodies 208L and 208R biases the front links 20L and 20R outward and biases the upper link 16 inward together with the disc springs 206L and 206R, which are also elastic members, thereby stabilizing the position of the upper link 16 in the left-right direction and suppressing rattling of the first shaft 24 in the axial direction. As described above, with the elastic bodies 208L and 208R of the present embodiment, rattling of the upper link 16 in the radial direction and the axial direction can be effectively suppressed.

Although the pair of left and right elastic bodies 208L and 208R do not inhibit the rotation of the upper link 16 around the first shaft 24, the cross-sectional shape of the annular elastic bodies 208L and 208R surrounding the outer periphery of the first shaft 24 may be a shape including irregularities such as a substantially X shape in order to achieve more lubricating rotation. In addition, a lubricant such as lubricating oil or grease may be added to the irregularities, and the surfaces of the elastic bodies 208L and 208R and/or the first shaft 24 may be coated with a low-friction fluororesin. The cross-sectional shape of the elastic bodies 208L and 208R is not limited to the substantially X shape, and may be a circular shape, an elliptical shape, a rectangular shape, a rhombus shape, a polygonal shape, a star shape, or the like.

Since the above description of the first shaft 24, which is an upper rotation shaft member, also applies to the third shaft 28, which is a lower rotation shaft member, redundant description is omitted (the "upper link 16" in the above description can be replaced with the "lower link 18"). Regarding the first shaft 24, the left and right front links 20L and 20R and the first shaft 24 are firmly fixed and integrated by the left and right fixing members 202L and 202R. Regarding the third shaft 28, the left and right front links 20L and 20R and the third shaft 28 are firmly fixed and integrated by the left and right fixing members 209L and 209R. That is, a highly rigid integrated structure in which the left and right front links 20L and 20R, the first shaft 24, and the third shaft 28 are firmly fixed is achieved.

When the rigidity of the rectangular structure formed by the left and right front links 20L and 20R, the first shaft 24, and the third shaft 28 is low, the rectangular structure is distorted in a parallelogram shape by an external force. In this case, the bearings (207L, 207R, etc.) locally come into strong contact with the rotation shaft members (the first shaft 24, etc.), so that the wear of the bearings progresses quickly. In contrast, according to the present embodiment, since the rigidity of the rectangular structure formed by the left and right front links 20L and 20R, the first shaft 24, and the third shaft 28 can be increased, wear of the bearings can be suppressed. In addition, as described above, even if the bearings wear, thanks to the elastic deformation in the radial direction and the axial direction of the elastic bodies 208L and 208R, rattling in both directions can be suppressed, so that comfortable walking can be achieved without giving discomfort to the user.

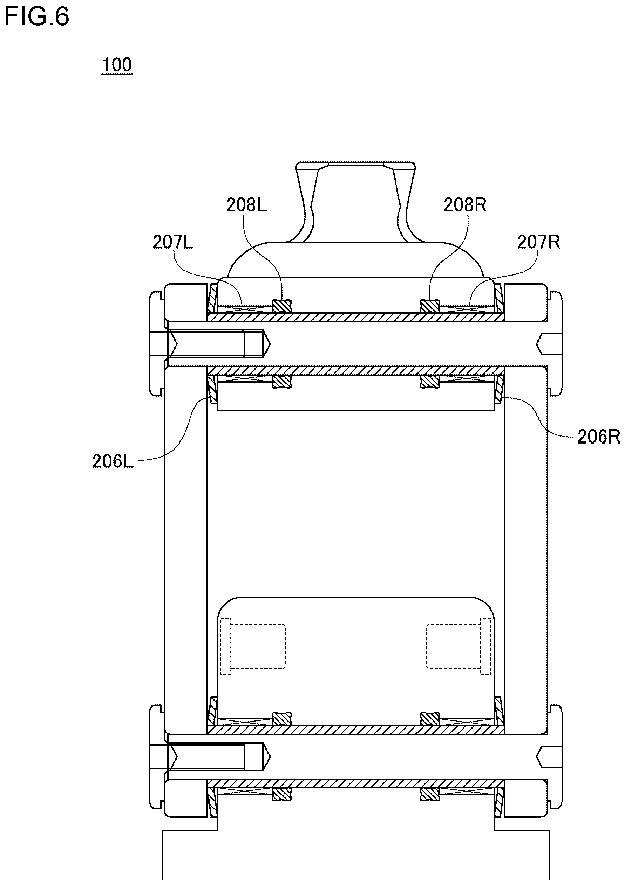

Fig. 6 is a schematic cross-sectional view of a multi-articulated link knee joint 100 according to a second configuration example. As compared with the first configuration example in Fig. 5, the positions in the left-right direction of the pair of left and right bearings 207L and 207R and the pair of left and right elastic bodies 208L and 208R are reversed. That is, the left first bearing 207L is provided closer to the left end portion of the first shaft 24 than the left first elastic body 208L, and the right second bearing 207R is provided closer to the right end portion of the first shaft 24 than the right second elastic body 208R. Instead of the pair of left and right elastic bodies 208L and 208R, one elastic body may be provided at the center in the axial direction of the first shaft 24.

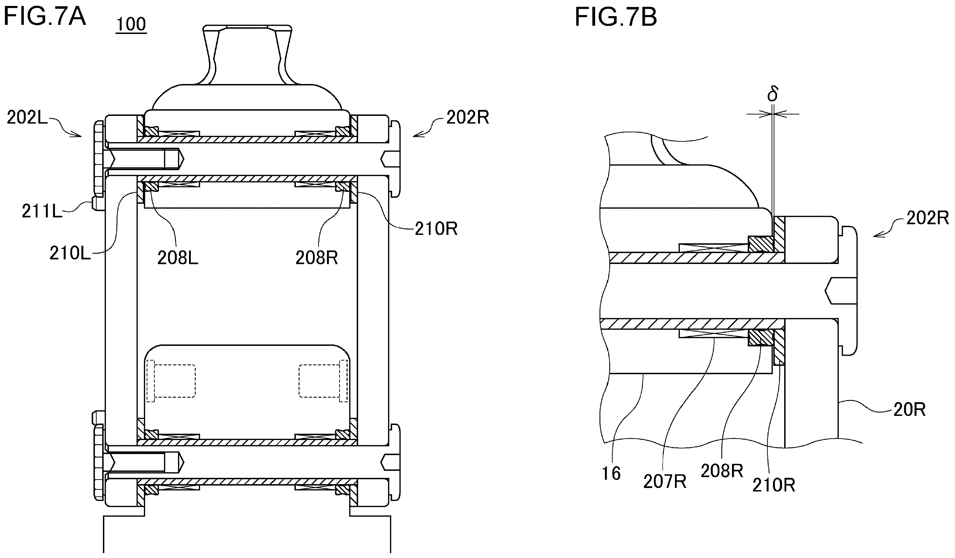

Figs. 7A and 7B are schematic cross-sectional views of a multi-articulated link knee joint 100 according to a third configuration example. As compared with the first configuration example of Fig. 5, a pair of left and right plastic flat washers 210L and 210R are provided as end members instead of the pair of left and right disc springs 206L and 206R, and a positioning screw 211L for fixing the left fixing member 202L at a desired fastening position is provided. As illustrated in the enlarged view in Fig. 7B, the elastic body 208R that elastically deforms not only in the radial direction but also in the axial direction protrudes outward (right side) from the right end of the upper link 16 and contacts the inner (left side) surface of the flat washer 210R to bias outward. Although the plastic flat washer 210R may be worn by the relative rotation of the upper link 16 and the front link 20R, the protruding amount δ of the elastic body 208R that elastically deforms in the axial direction increases to compensate for the wear, so that the contact state of the upper link 16 and the front link 20R in the axial direction via the elastic body 208R and the flat washer 210R is favorably maintained. Therefore, rattling in the axial direction can be suppressed regardless of wear of the flat washer 210R. The protruding amount δ of each of the elastic bodies 208L and 208R when the flat washers 210L and 210R are not worn is, for example, between 0.2 mm and 0.3 mm, and is determined by the length in the left-right direction of the rotation shaft member 24, the upper link 16, and the flat washers 210L and 210R. Specifically, 2δ = length of the rotation shaft member 24 in the left-right direction - (length of the upper link 16 in the left-right direction + length of the flat washer 210L in the left-right direction + length of the flat washer 210R in the left-right direction).

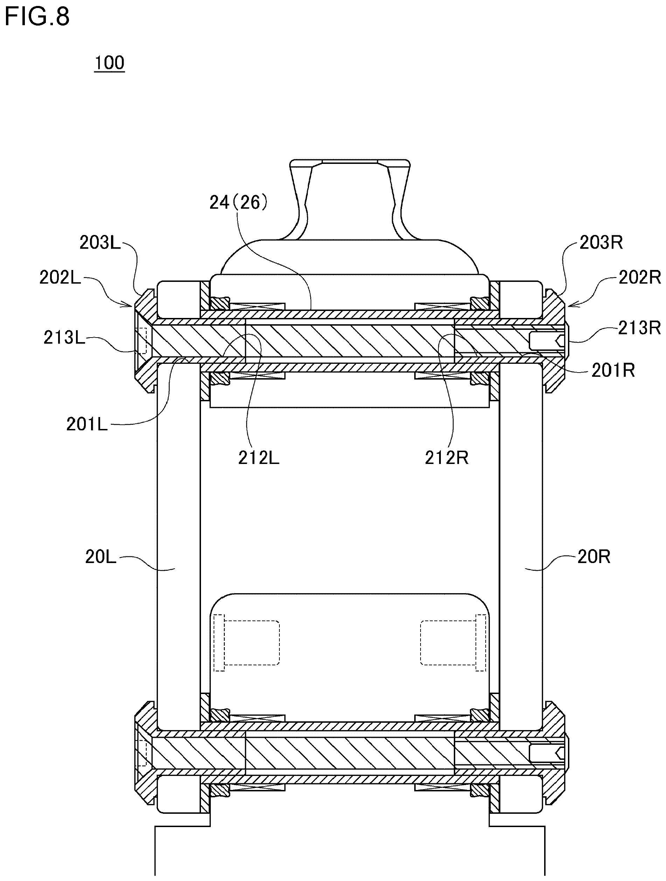

Fig. 8 is a schematic cross-sectional view of a multi-articulated link knee joint 100 according to a fourth configuration example. The configuration of the pair of left and right fixing members 202L and 202R is different from that of the third configuration example of Figs. 7A and 7B. The left fixing member 202L is a nut member including a locking portion 203L provided on the proximal end side (left end side) and having an area larger than the area of the through hole 201L of the front link 20L, and a female screw portion 212L extending from the locking portion 203L to the distal end side (right end side). The right fixing member 202R is a nut member including a locking portion 203R provided on the proximal end side (right end side) and having an area larger than the area of the through hole 201R of the front link 20R, and a female screw portion 212R extending from the locking portion 203R to the distal end side (left end side). A long bolt member 213L is inserted from the left side so as to pass through the pair of left and right nut members 202L and 202R in the left-right direction, and is engaged with the respective female screw portions 212L, 212R. In this manner, the left fixing member 202L and the right fixing member 202R are connected to each other inside the first shaft 24 formed in a tubular shape by engagement with the bolt member 213L. An anti-loosening bolt 213R for preventing loosening is attached to the distal end portion (right end portion) of the bolt member 213L. In addition to or instead of the anti-loosening bolt 213R, an adhesive or the like may be used for preventing loosening.

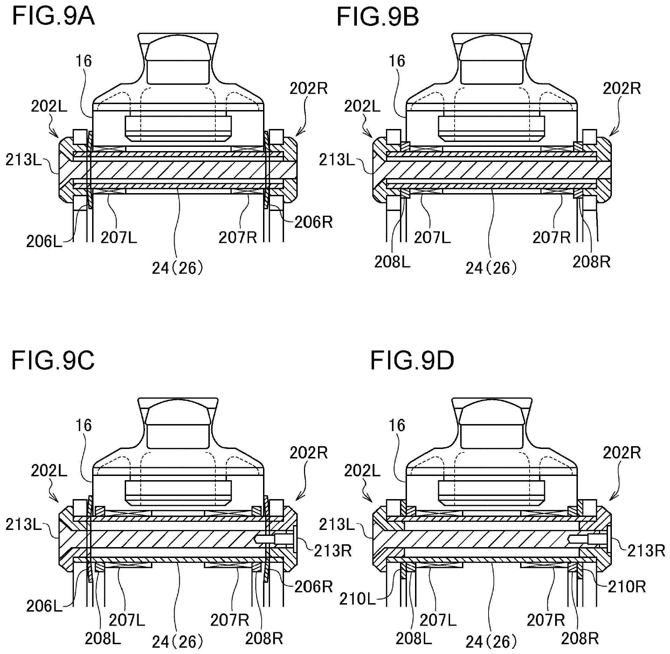

Figs. 9A to 9D schematically illustrate modification examples of the fourth configuration example of Fig. 8. In Fig. 8, the pair of left and right fixing members 202L and 202R is inserted inside the first shaft 24, but in Fig. 9A, the first shaft 24 is accommodated inside the pair of left and right fixing members 202L and 202R. Further, the pair of left and right fixing members 202L and 202R press the disc springs 206L and 206R inward in the left-right direction at the respective distal end surfaces. In Fig. 9B, similarly to Fig. 9A, the first shaft 24 is accommodated inside the pair of left and right fixing members 202L and 202R, and elastic bodies 208L and 208R that are elastically deformable in the radial direction and the axial direction are provided instead of the disc springs 206L and 206R. In Fig. 9C, the first shaft 24 is provided outside the pair of left and right fixing members 202L and 202R. The pair of left and right fixing members 202L and 202R press the disc springs 206L and 206R inward in the left-right direction, and are provided with the elastic bodies 208L and 208R that are in contact with the outer periphery of the first shaft 24 and are elastically deformable in the radial direction. In Fig. 9D, similarly to Fig. 9C, the first shaft 24 is provided outside the pair of left and right fixing members 202L and 202R. The flat washers 210L and 210R described with reference to Figs. 7A and 7B are provided instead of the disc springs 206L and 206R illustrated in Fig. 9C. Similarly to Figs. 7A and 7B, the elastic bodies 208L and 208R that elastically deform not only in the radial direction but also in the axial direction suppress rattling of the front link 20 and the upper link 16 in the axial direction by biasing the flat washers 210L and 210R outward.

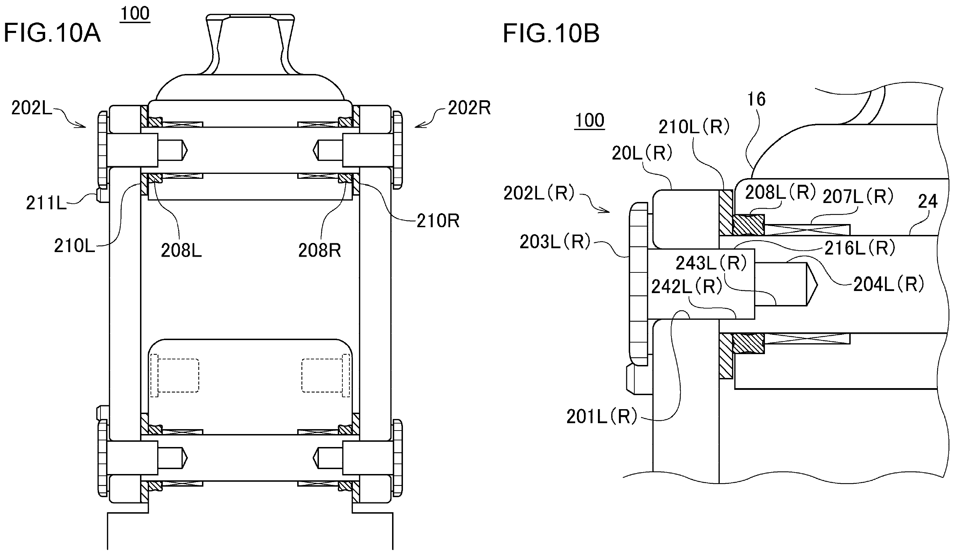

Figs. 10A and 10B are schematic cross-sectional views of a multi-articulated link knee joint 100 according to a fifth configuration example. Fig. 10B is an enlarged view of the periphery of the fixing member 202L in Fig. 10A. The configurations of the rotation shaft member 24 and the pair of left and right fixing members 202L and 202R are different from those of the third configuration example of Figs. 7A and 7B. The rotation shaft member 24 is a substantially columnar member, and includes large-diameter first recesses 242L and 242R bored from both left and right bottom surfaces, and small-diameter second recesses 243L and 243R bored from bottom surfaces of the first recesses 242L and 242R. The cross-sectional shapes of the first recesses 242L and 242R taken along a plane perpendicular to the rotation shaft of the rotation shaft member 24 are substantially equal to the cross-sectional shapes of the through holes 201L and 201R of the front links 20L and 20R. The cross-sectional shapes of the second recesses 243L and 243R taken along a plane perpendicular to the rotation shaft of the rotation shaft member 24 are smaller than the cross-sectional shapes of the first recesses 242L and 242R. A female screw is formed on the inner peripheral surface of each of the second recesses 243L and 243R.

The fixing members 202L and 202R include large-diameter parts 216L and 216R having large diameters and extending inward or toward the distal end side from the locking portions 203L and 203R, and male screw portions 204L and 204R having small diameters and extending inward or toward the distal end side from the large-diameter parts 216L and 216R. The cross-sectional shapes of the large-diameter parts 216L and 216R taken along a plane perpendicular to the rotation shaft of the rotation shaft member 24 are substantially equal to the cross-sectional shapes of the through holes 201L and 201R of the front links 20L and 20R and the first recesses 242L and 242R. The lengths of the large-diameter parts 216L and 216R in the axial direction or the left-right direction are larger than the lengths of the front links 20L and 20R. Therefore, the distal end portions of the large-diameter parts 216L and 216R inserted into the through holes 201L and 201R of the front links 20L and 20R protrude inward from the front links 20L and 20R and are fitted into the first recesses 242L and 242R of the rotation shaft member 24. The cross-sectional shapes of the male screw portions 204L and 204R taken along a plane perpendicular to the rotation shaft of the rotation shaft member 24 are substantially equal to the cross-sectional shapes of the second recesses 243L and 243R of the rotation shaft member 24. Therefore, the male screw portions 204L and 204R are inserted into the second recesses 243L and 243R, and are engaged with the female screws formed on the inner peripheral surfaces thereof.

Here, when the left fixing member 202L and the right fixing member 202R are firmly fastened, the left front link 20L is strongly pressed and fixed to the left end surface of the rotation shaft member 24 by the locking portion 203L of the left fixing member 202L, and the right front link 20R is strongly pressed and fixed to the right end surface of the rotation shaft member 24 by the locking portion 203R of the right fixing member 202R. In this manner, the left and right front links 20L and 20R and the rotation shaft member 24 are firmly fixed and integrated by the left fixing member 202L and the right fixing member 202R serving as fixing members.

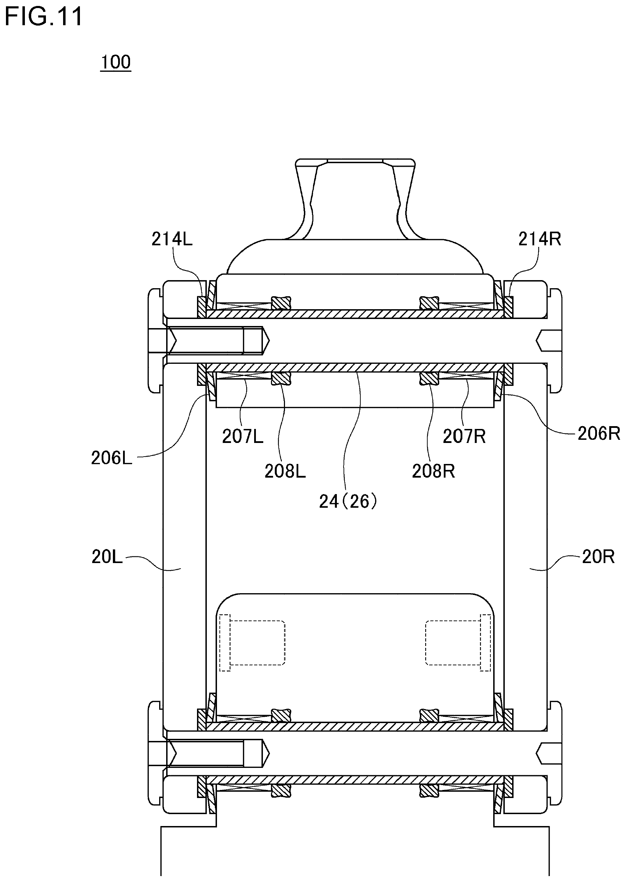

Fig. 11 is a schematic cross-sectional view of a multi-articulated link knee joint 100 according to a sixth configuration example. As compared with the first configuration example in Fig. 5, in the left and right front links 20L and 20R mostly formed of an aluminum alloy or the like, washers 214L and 214R serving as end members formed of a high strength and high hardness material such as a titanium alloy, stainless steel, hardened steel, or the like are embedded in the contact portions with both end surfaces of the first shaft 24, so that the rigidity of the multi-articulated link knee joint 100 is increased. When the washers 214L and 214R receive strong surface pressure from both end surfaces of the first shaft 24, the surface pressure applied to the front links 20L and 20R is reduced, so that it is possible to prevent deformation and deterioration of the front links 20L and 20R having lower strength and lower hardness than those of the washers 214L and 214R, and it is possible to maintain the structure with high rigidity according to the present embodiment over a long period of time. As the hardened steel, martensitic stainless steel such as SUS440C, which hardly generates rust, and stainless steel subjected to an age hardening heat treatment such as SUS630 are suitable. The stainless steel may be subjected to a surface hardening treatment such as a nitriding treatment. Although the first shaft 24 is also formed of a high-strength material such as stainless steel or a titanium alloy, when the first shaft 24 and the washers 214L and 214R in contact with each other are formed of a material having the same composition, there is a possibility that electrical corrosion is promoted, and thus, it is preferable that both have different compositions.

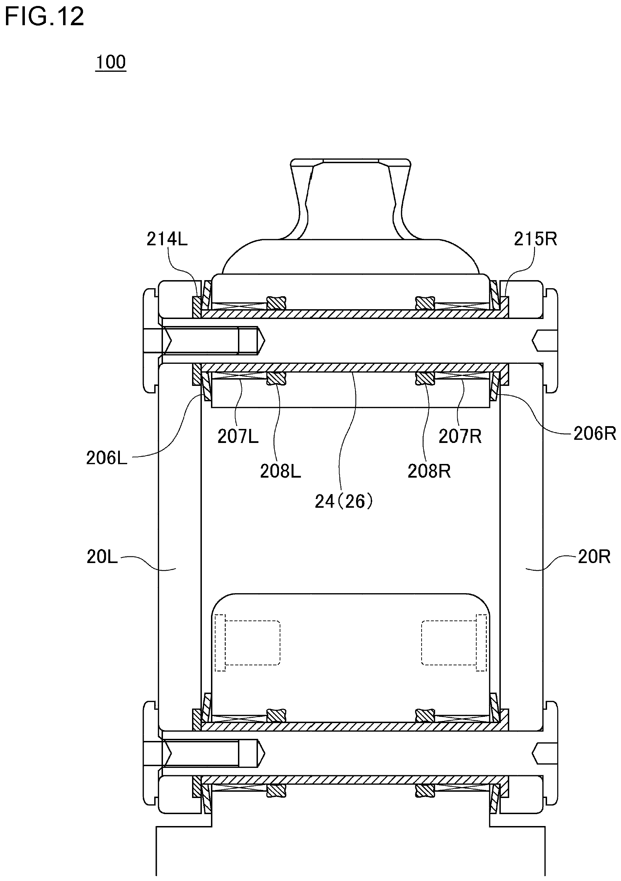

Fig. 12 is a schematic cross-sectional view of a multi-articulated link knee joint 100 according to a seventh configuration example. As compared with the sixth configuration example in Fig. 11, a radially protruding flange 215R is provided at the right end portion of the first shaft 24, and functions to increase rigidity similarly to the right washer 214R in Fig. 11. By providing the flange 215R, it is possible to effectively suppress twisting of the first shaft 24.

As above, the present invention has been described based on the embodiments. The embodiment is intended to be illustrative only and it will be understood by those skilled in the art that various modifications to constituting elements and processes could be developed and that such modifications are also within the scope of the present invention.

In the embodiments, the multi-articulated link knee joint 100 including the plurality of rotation shaft members 24, 26, 28, and 30 and the plurality of front and rear links 20L, 20R, 22L, and 22R has been described, but it is sufficient that at least one rotation shaft member is provided, and the front and rear links are not necessarily required. Therefore, the present invention is also applicable to an artificial knee joint having a uniaxial configuration in which the thigh joint part 32 and the lower leg part 12 are simply connected by one rotation shaft member.

In the embodiments, the multi-articulated link knee joint 100 including the thigh joint part 32 as the first member and the lower leg part 12 as the second member has been described, but the present invention is also applicable to any joint other than the knee. For example, the present invention can also be applied to an ankle joint device including a lower leg part 12 as a first member and a foot part as a second member, an elbow joint device including an upper arm part as a first member and a forearm part as a second member, and a wrist joint device including a forearm part as a first member and a hand part as a second member.

The functional configuration of each device described in the embodiments can be achieved by hardware resources or software resources or a cooperation of hardware resources and software resources. Processors, ROMs, RAMs, and other LSIs can be used as hardware resources. Programs such as operating systems and applications can be used as software resources.

Among the embodiments disclosed in the present specification, a configuration including a plurality of objects may integrate the plurality of objects, and conversely, a configuration including one object can be divided into a plurality of objects. The present invention may be configured to achieve an object of the invention regardless of whether or not the present invention is integrated.

Among the embodiments disclosed in the present specification in which a plurality of functions are distributed, some or all of the plurality of functions may be aggregated. Conversely, an aggregation of a plurality of functions may be distributed in part of in the entirety. Regardless of whether functions are aggregated or distributed, the functions may be configured to achieve the purpose of the invention.

## Claims

1. An artificial knee joint comprising:
a thigh joint part (32) provided on a thigh part side;
a lower leg part (12) rotatably connected to the thigh joint part (32) via at least one rotation shaft member;
a bearing that rotatably supports the rotation shaft member; and
an elastic body that is in contact with an outer peripheral surface of the rotation shaft member orthogonal to a rotation shaft, has a longer length from the rotation shaft than the bearing in a direction orthogonal to the rotation shaft, and is elastically deformable in a radial direction of the rotation shaft member.

2. The artificial knee joint according to claim 1, wherein the elastic body includes a first elastic body (208L) provided closer to a first end portion side of the rotation shaft member than the bearing and a second elastic body (208R) provided closer to a second end portion side of the rotation shaft member than the bearing.

3. The artificial knee joint according to claim 2, wherein the first elastic body (208L) is provided in contact with an outer periphery of a first end portion of the rotation shaft member, and the second elastic body (208R) is provided in contact with an outer periphery of a second end portion of the rotation shaft member.

4. The artificial knee joint according to any of claims 1 to 3, wherein the elastic body is elastically deformable in an axial direction of the rotation shaft member in contact with an end member provided at an end portion of the rotation shaft member.

5. The artificial knee joint according to claim 1, wherein the bearing includes a first bearing (207L) provided closer to a first end portion side of the rotation shaft member than the elastic body and a second bearing (207R) provided closer to a second end portion side of the rotation shaft member than the elastic body.

6. The artificial knee joint according to any of claims 1 to 5, wherein a washer (214) or a disc spring is provided as an end member on an outer periphery of an end portion of the rotation shaft member.

7. The artificial knee joint according to any of claims 1 to 6, wherein the elastic body is formed in an annular shape surrounding an outer periphery of the rotation shaft member and has a cross-sectional shape including irregularities, the cross-sectional shape being taken along a plane including the rotation shaft of the rotation shaft member.

8. The artificial knee joint according to claim 7, wherein a lubricant is added to the irregularities.

9. The artificial knee joint according to any of claims 1 to 8, wherein the bearing is a plain bearing.

10. The artificial knee joint according to any of claims 1 to 9, wherein
the thigh joint part (32) and the lower leg part (12) are rotatably connected via the rotation shaft member and a pair of links provided at both end portions of the rotation shaft member,
the artificial knee joint further comprises a fixing member that presses and fixes the pair of links against both end portions of the rotation shaft member, and
at least one of the thigh joint part (32) and the lower leg part (12) is rotatably provided on an outer periphery of the rotation shaft member via the bearing.

11. The artificial knee joint according to claim 1, comprising:
a thigh joint part (32) provided on a thigh part side;
at least one rotation shaft member rotatably provided around a rotation shaft;
a lower leg part (12) rotatably connected to the thigh joint part (32) via the rotation shaft member and a pair of links provided at both end portions of the rotation shaft member; and
a fixing member that presses and fixes the pair of links against both end portions of the rotation shaft member, wherein
at least one of the thigh joint part (32) and the lower leg part (12) is rotatably provided on an outer periphery of the rotation shaft member.

12. The artificial knee joint according to claim 10 or 11, wherein
the fixing member includes a first fixing member provided on a first end portion side of the rotation shaft member and a second fixing member provided on a second end portion side of the rotation shaft member, and
the first fixing member and the second fixing member are connected to each other inside the rotation shaft member.

13. The artificial knee joint according to any of claims 10 to 12, wherein a material of the rotation shaft member is harder than a material of the pair of links at a position pressed against both end portions of the rotation shaft member.

14. The artificial knee joint according to any of claims 10 to 13, wherein a washer or a disc spring is provided as an end member between both end portions of the rotation shaft member and the pair of links.

15. The artificial knee joint according to any of claims 10 to 14, wherein an end portion of the rotation shaft member is provided with a radially protruding flange.

16. The artificial knee joint according to any of claims 1 to 15, wherein the thigh joint part (32) and the lower leg part (12) are rotatably connected via a plurality of the rotation shaft members and a pair of links connecting both end portions to each other of the plurality of the rotation shaft members.
